# FASCICULE DE BREVET EUROPEEN

(11) **EP 0 750 635 B1**
(45) Date de publication et mention de la délivrance du brevet: **02.12.1998**
(21) Numéro de dépôt: 95912309.2
(22) Date de dépôt: 14.03.1995
(51) Int. Cl.: C07K 14/47, A61K 38/17

(54) **PEPTIDES POUR INHIBER LA LIBERATION DE PEPSINE**
PEPTIDE FÜR DIE INHIBITION DER PEPSINFREISETZUNG
PEPTIDES INHIBITING THE RELEASE OF PEPSINE

(30) Priorité: 16.03.1994 GB 9405162
(43) Date de publication de la demande: 02.01.1997
(73) Titulaire: SOCIETE DE CONSEILS DE RECHERCHES ET D'APPLICATIONS SCIENTIFIQUES (S.C.R.A.S.), 75016 Paris (FR); INSTITUT NATIONAL DE LA SANTE ET DE LA RECHERCHE MEDICALE (INSERM), 75654 Paris Cédex 13 (FR)
(72) Inventeur: DESCROIX-VAGNE, Monique, F-69450 Saint-Cyr-au-Mont-d'Or (FR); PANSU, Danielle, F-39570 Lons-le-Saunier (FR); TARRADE, Thierry, F-91190 Gif-sur-Yvette (FR)
(74) Mandataire: Bourgouin, André
(86) Numéro de dépôt international: FR9500296
(87) Numéro de publication internationale: WO9525123

(56) Documents cités:
- WO-A-89/06241
- FR-A- 2 298 334
- GASTROENTEROLOGY (1992), 103(5), 1568-73 CODEN: GASTAB;ISSN: 0016-5085, CHARPIN, GHISLAINE ET AL 'Effect of sorbin on duodenal absorption of water and electrolytes in the rat'
- DATABASE WPI Section Ch, Week 4691 Derwent Publications Ltd., London, GB; Class B04, AN 91-337300 & PT-A-93 320 (TULANE E FUND ADMINISTRA) , 15 Octobre 1991

## Description

L'invention a pour objet des peptides capables d'inhiber la libération de pepsine, les dérivés de substitution et les sels de tels peptides et des compositions pharmaceutiques contenant ces peptides. Les peptides peuvent être utilisés dans le traitement de maladies en relation avec la libération de pepsine et, plus particulièrement, pour le traitement des ulcères ou de l'oesophagite.

Récemment, un nouveau peptide a été isolé de l'intestin de porc ; ce peptide, appelé sorbine, compte 153 acides aminés naturels (WO 89/06241). La sorbine ainsi que ces fragments peptidiques de la partie C-terminale de la sorbine (contenant, au plus, 40 acides aminés), sont capables de provoquer un accroissement du processus d'absorption par la muqueuse. Cependant, la modification de ces fragments peptidiques par insertion d'au moins un résidu d'acide aminé de configuration D, confère, de façon inattendue, une autre activité biologique à ces analogues peptidiques modifiés : ils inhibent la libération de pepsine, activité biologique que les peptides non modifiés ne présentent pas.

Cette activité est particulièrement intéressante dans certaines circonstances. En effet, les mécanismes généraux de la sécrétion gastrique chez les mammifères sont bien connus actuellement. La digestion gastrique est donc la résultante de l'action d'enzymes, de l'acide chlorhydrique et de pepsine. La pepsine est une protéine ; c'est aussi, avec la gastrine, l'un des constituants principaux du suc gastrique. Son rôle physiologique majeur est l'initiation de la digestion de protéines. En contrepartie, de nombreuses études ont montré le rôle significatif de la pepsine dans la formation d'ulcère. Par conséquent, dans certaines circonstances, il peut être souhaitable d'inhiber au moins partiellement la libération de pepsine.

L'invention a donc pour objet un peptide de formule générale I

A₁―A₂―A₃―X I

dans laquelle
A₁ représente le résidu L-Thr ou D-Thr ; ou l'une des séquences suivantes dans laquelle au moins un résidu d'acide aminé peut être de configuration D : 
Val-Thr,
Pro-Val-Thr,
Arg-Pro-Val-Thr,
Glu-Arg-Pro-Val-Thr,
His-Glu-Arg-Pro-Val-Thr,
Gln-His-Glu-Arg-Pro-Val-Thr,
Leu-Gln-His-Glu-Arg-Pro-Val-Thr,
Ile-Leu-Gln-His-Glu-Arg-Pro-Val-Thr,
Ser-Ile-Leu-Gln-His-Glu-Arg-Pro-Val-Thr,
Ser-Ser-Ile-Leu-Gln-His-Glu-Arg-Pro-Val-Thr,
Lys-Ser-Ser-Ile-Leu-Gln-His-Glu-Arg-Pro-Val-Thr,
Gly-Lys-Ser-Ser-Ile-Leu-Gln-His-Glu-Arg-Pro-Val-Thr,
Pro-Gly-Lys-Ser-Ser-Ile-Leu-Gln-His-Glu-Arg-Pro-Val-Thr ou
Glu-Pro-Gly-Lys-Ser-Ser-Ile-Leu-Gln-His-Glu-Arg-Pro-Val-Thr;
A₂ représente la séquence Lys-Pro-Gln-Ala dans laquelle au moins un résidu d'acide aminé peut être de configuration D ;
A₃ représente une liaison covalente ou la séquence peptidique -Gly-A₄-A₅ dans laquelle A₄ et A₅ représentent, indépendamment, chacun, un résidu d'acide aminé basique ; et
X représente le groupement hydroxy ; amino ; alkylamino, ledit peptide de formule I caractérisé en ce qu'il contient au moins un résidu d'acide aminé de configuration D.

L'invention a également pour objet des dérivés de substitution des peptides de formule générale I dans lesquels un ou plusieurs résidus d'acide aminé est substitué par un ou plusieurs groupes protecteurs de peptides couramment utilisés dans la chimie des peptides à usage biologique ; lorsque plusieurs groupes protecteurs sont utilisés, ces derniers ne sont pas nécessairement les mêmes. De préférence, les groupes protecteurs sont sélectionnés parmi les groupes alkyl inférieur tel que méthyl ou ter-butyl; phényl ; benzyl ou benzyl substitué tel que trimethoxybenzyl ; 2-chlorobenzyloxycarbonyl ; 9-fluorenylmethyloxycarbonyl ; ter-butyloxycarbonyl ; acétyl ; sulfonyl ; et phosphoryl.

L'invention a également pour objet des peptides contenant la séquence d'acide aminé A₁-A₂-A₃, dans laquelle A₁, A₂ et A₃ sont tels que définis ci-dessus.

L'invention concerne également les sels pharmaceutiquement acceptables des peptides tels que définis ci-dessus. Ces sels peuvent être obtenus avec des acides organiques tels que l'acide acétique, lactique, pamoïque, maléique, citrique, malique, ascorbique, benzoïque, salicylique, succinique, méthylsulfonique, toluènesulfonique, des acides minéraux tels que l'acide chlorhydrique, sulfurique ou phosphorique, ou bien des acides polymériques tels que l'acide tannique ou la carboxyméthyl cellulose.

A₄ et A₅ représentent, indépendamment, quand ils sont présents dans les peptides selon l'invention, des résidus d'acide aminé basique, de configuration D ou L. De préférence, A₄ et A₅ représentent, indépendamment, le résidu d'acide aminé Lys, D-Lys, Arg ou D-Arg.

Tel que mentionné ci-dessus, les peptides selon l'invention contiennent un ou plusieurs résidus d'acide aminé de configuration D. L'invention vise plus particulièrement les peptides contenant un résidu d'acide aminé de configuration D, lesdits peptides pouvant être substitués par des groupes protecteurs tels que définis dans la présente invention. On peut citer en exemple les peptides suivants

Thr-Lys-Pro-Gln-D-Ala-NH2,
Thr-Lys-Pro-Gln-D-Ala-Gly-Lys-Lys-NH2,
Thr-(acétyl)Lys-Pro-Gln-D-Ala-NH2,
Pro-Val-Thr-Lys-Pro-Gln-D-Ala-NH2,
Pro-D-Val-Thr-Lys-Pro-Gln-Ala-NH2,
Pro-Val-Thr-Lys-Pro-Gln-Ala-Gly-Arg-D-Arg,
Pro-Val-Thr-(acétyl)Lys-Pro-Gln-D-Ala-NH2,
D-Pro-Val-Thr-Lys-Pro-Gln-Ala-NH2,
His-Glu-Arg-Pro-Val-Thr-Lys-Pro-Gln-D-Ala-NH2,
Ile-Leu-Gln-His-Glu-Arg-Pro-Val-Thr-Lys-Pro-Gln-D-Ala-NH2 et
Glu-Pro-Gly-Lys-Ser-Ser-Ile-Leu-Gln-His-Glu-Arg-Pro-Val-Thr-Lys-Pro-Gln-D-Ala-
NH2.

Le résidu d'acide aminé de configuration D se situe de préférence sur l'extrémité C-terminale ou N-terminale. Les peptides préférés qui présentent un résidu d'acide aminé de configuration D en position C-terminale sont les peptides dans lesquels A₂ représente Lys-Pro-Gln-D-Ala et A₃ représente une liaison covalente. Les peptides préférés qui présentent un résidu d'acide aminé de configuration D en position N-terminale, sont les peptides dans lesquels A₁ représente D-Thr ou une séquence telle que définie ci-dessus dans laquelle le résidu d'acide aminé en position N-terminale de cette séquence est de configuration D.

L'invention vise plus particulièrement, également, les peptides qui présentent deux résidus d'acide aminé de configuration D, lesdits peptides pouvant être substitués par des groupes protecteurs tels que définis dans la présente invention. On peut citer en exemple les peptides suivant

D-Pro-Val-Thr-Lys-Pro-Gln-D-Ala-NH2,
D-Pro-Val-Thr-Lys-Pro-Gln-Ala-Gly-D-Lys-Lys-NH2,
Pro-D-Val-Thr-Lys-Pro-Gln-D-Ala-NH2 et
D-Pro-Val-Thr-(acétyl)Lys-Pro-Gln-D-Ala-NH2.

De préférence, les peptides contenant deux résidus d'acide aminé de configuration D présentent un résidu d'acide aminé de configuration D en position C-terminale. Le deuxième résidu d'acide aminé peut se situer n'importe où sur la chaîne peptidique, mais de préférence en position N-terminale du peptide. Les peptides préférés sont les peptides dans lesquels A₁ représente D-Thr ou une séquence telle que définie ci-dessus dans laquelle le résidu d'acide aminé en position N-terminale de cette séquence est de configuration D, A₂ représente Lys-Pro-GIn-D-Ala et A₃ représente une liaison covalente. De tels peptides sont, de préférence, substitués par un ou des groupes protecteurs, et plus particulièrement par le groupe protecteur acétyl sur le résidu Lys.

Les peptides selon l'invention peuvent être préparés selon l'une des méthodes classiques connues dans le domaine des synthèses peptidiques. Par exemple, la synthèse peut avantageusement s'effectuer en phase solide selon le schéma suivant : la formation de la chaîne peptidique commence par la fixation du premier acide aminé C-terminal de la chaîne, sur une résine, par l'intermédiaire de son groupe carboxylique ; sa fonction amine est protégée avec un groupe protecteur tel que t-butyloxycarbonyle (Boc). Après fixation du premier acide aminé C-terminal, la fonction amine est déprotégée par lavage de la résine par un acide. Dans le cas d'une protection avec le groupe Boc, la déprotection peut se faire par lavage avec l'acide trifluoroacétique. Le second acide aminé, dont la fonction amine est protégée, est couplé, par l'intermédiaire de sa fonction carboxyle, à la fonction amine déprotégée du premier acide aminé C-terminal de la chaîne. Le couplage s'effectue de préférence en présence d'un agent de couplage tel que le dicyclohexylcarbodiimide ou le diisopropylcarbodiimide. La chaîne peptidique ainsi formée comprend deux acides aminés dont la jonction amine terminale est protégée. Comme précédemment, cette fonction amine terminale est déprotégée et on peut alors procéder à la fixation du troisième acide aminé. La chaîne peptidique souhaitée est ainsi obtenue par fixation des acides aminés, les uns après les autres. Après élimination de tous les groupes protecteurs, le peptide est détaché de la résine.

La synthèse d'un peptide de l'invention, à savoir Pro-Val-Thr-Lys-Pro-Gln-D-Ala-NH₂, est décrite brièvement ci-dessous. D'autres peptides de l'invention peuvent être préparés par des modifications appropriées de la synthèse peptidique ci-dessous qui sont à la portée de l'homme du métier dans ce domaine.

La synthèse est effectuée en phase solide, à température ambiante. Le mode opératoire utilisé comprend les étapes suivantes : déprotection, neutralisation et couplage. La résine utilisée est de type polystyrène réticulé à 1 % de divinyl benzène (résine Merrifield). La fixation du Boc-D-Ala sur la résine Merrifield se fait en présence de carbonate de césium dans le toluène et le diméthylformamide (DMF). La fonction amine terminale des acides aminés utilisés est protégée par le groupement Boc. Ces groupements Boc sont déplacés par l'acide trifluoroacétique, suivi de plusieurs lavages avec du chlorure de méthylène et de l'isopropanol. Les groupements aminés sont neutralisés avec de la triéthylamine suivi de plusieurs lavages. La thréonine et la valine sont transformées avant couplage en un ester de l'hydroxybenzotriazole en présence de diisopropylcarbodiimide (DIPCDI), pour la glutamine, l'ester de l'hydroxybenzotriazole se fait directement dans le réacteur. La lysine et les deux prolines sont transformées en anhydride symétrique avant couplage. Dans tous les cas, le couplage est effectué en présence de diisopropyléthylamine. La chaîne latérale de la lysine est protégée par un groupement Fmoc, alors que celle de la thréonine n'est pas protégée. Le dernier couplage terminé, le déplacement du groupement Fmoc est obtenu par la piperidine dans le diméthylformamide ayant le déplacement du groupement Boc de la fonction amine N-terminale de la proline. Le peptide est obtenu par clivage de la résine, après traitement à l'ammoniac dans le mélange méthanol / DMF. Le produit brut, ainsi obtenu, est ensuite purifié.

L'invention vise également des compositions pharmaceutiques contenant, à titre de principe actif, une quantité efficace d'au moins un peptide de formule I tel que défini ci-dessus, un dérivé de substitution d'un tel peptide tel que défini ci-dessus, ou un peptide renfermant la séquence d'acide aminé A₁-A₂-A₃ telle que définie ci-dessus, en association avec un diluent ou support pharmaceutiquement acceptable.

Les peptides de l'invention peuvent être administrés par voies orale, intraveineuse, parentérale, sous-cutanée, intrapéritonéale ou intramusculaire.

La composition pharmaceutique peut se présenter sous la forme d'une gélule, d'un comprimé, d'un lyophilisat ou d'un liquide selon le mode d'administration choisi. La composition pharmaceutique peut également être sous la forme d'une formulation à libération prolongée.

Par voie orale, le peptide selon l'invention peut être administré, chez l'homme, à une dose de 5 à 100 µg/kg par jour.

Par voie intraveineuse ou sous-cutanée, un composé selon l'invention peut alors être administré, chez l'homme, à une dose dc 1 à 12 µg/kj, une à trois fois par jour. Chez l'animal, les composés selon l'invention sont retrouvés en quantité importante dans l'organisme, plusieurs jours après une administration aiguë, et plus particulièrement le peptide Pro-Val-Thr-Lys-Pro-Gln-D-Ala-NH₂ qui est retrouvé en quantité supérieure à 10 %.

### Toxicité

La toxicité sub-aiguë a été étudiée chez le rat et le chien. Suite à des administrations de doses allant jusqu'à 4000 µg/kg/j, aucun signe de toxicité et aucun signe évoquant un pouvoir mutagène n'ont été observés quatre semaines après l'administration. Chez l'homme, une injection sous-cutanée ou intraveineuse, à la dose de 200 µg/kg, n'entraîne aucune anomalie biologique, clinique ou pathologique.

### Pharmacologie

L'intérêt thérapeutique des composés de l'invention est déterminé par l'expérimentation suivante.

L'intensité de la réponse gastrique est mesurée en déterminant le volume de sécrétion gastrique induite.

Des chats sont opérés, sous anesthésie générale : l'intervention permet de diviser l'estomac en deux parties : la poche dite d'Heidenheim et la fistule gastrique. Ces deux poches sont dérivées vers l'extérieur, afin de récupérer les sécrétions d'acide chlorhydrique, de pepsine et de suc gastrique, à la fois pendant la phase basale puis après stimulation. Ce sont des chats qui ont des fistules chroniques : ils peuvent donc subir un certain nombre de tests chaque semaine et être leurs propres contrôles. La stimulation de la sécrétion de pepsine est obtenue par administration, chez les animaux en vie, de pentagastrine (PG) et du VIP (Vasoactive Intestinal Peptide), par perfusion pendant 2 heures, à raison de 2 et 4 µg/kg/h.

Une heure après la stimulation par la pentagastrine et le VIP, ces peptides sont ajoutés en perfusion, à la dose de 100 pmol/kg/h.

Le volume de suc gastrique est recueilli pendant les 30 minutes qui précèdent la perfusion, jusqu'à la fin de la perfusion.

Le dosage de pepsine du suc gastrique (le plus homogène possible) est évalué par une méthode spectrophotométrique protéolytique.

Les résultats obtenus lors de 9-12 expériences sont reportés dans les tableaux ci-dessous : la sécrétion de pepsine est exprimée en mg/15 minutes, moyenne de 2 périodes de 15 minutes par test pendant la sécrétion basale et moyenne de 6 périodes de 15 minutes pendant la sécrétion stimulée.

Certains peptides de l'invention, présentant au moins un résidu d'acide aminé sous forme D, sont comparés avec leurs analogues dont les résidus d'acides aminés sont tous de configuration L.

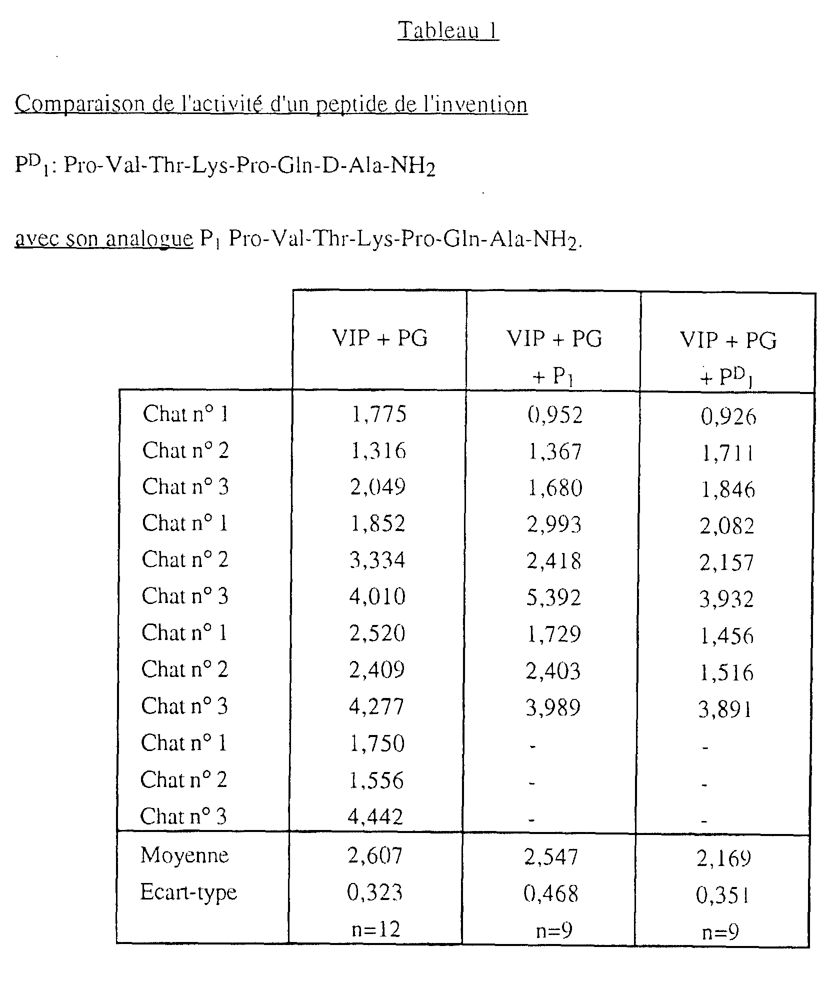

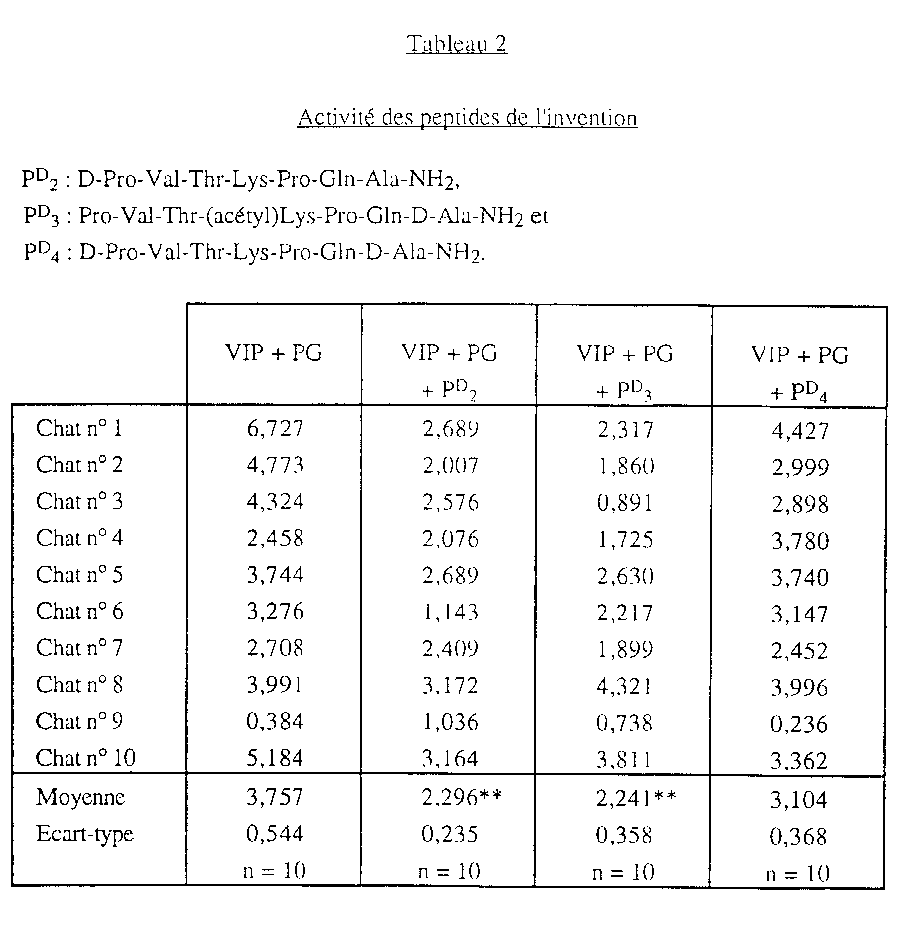

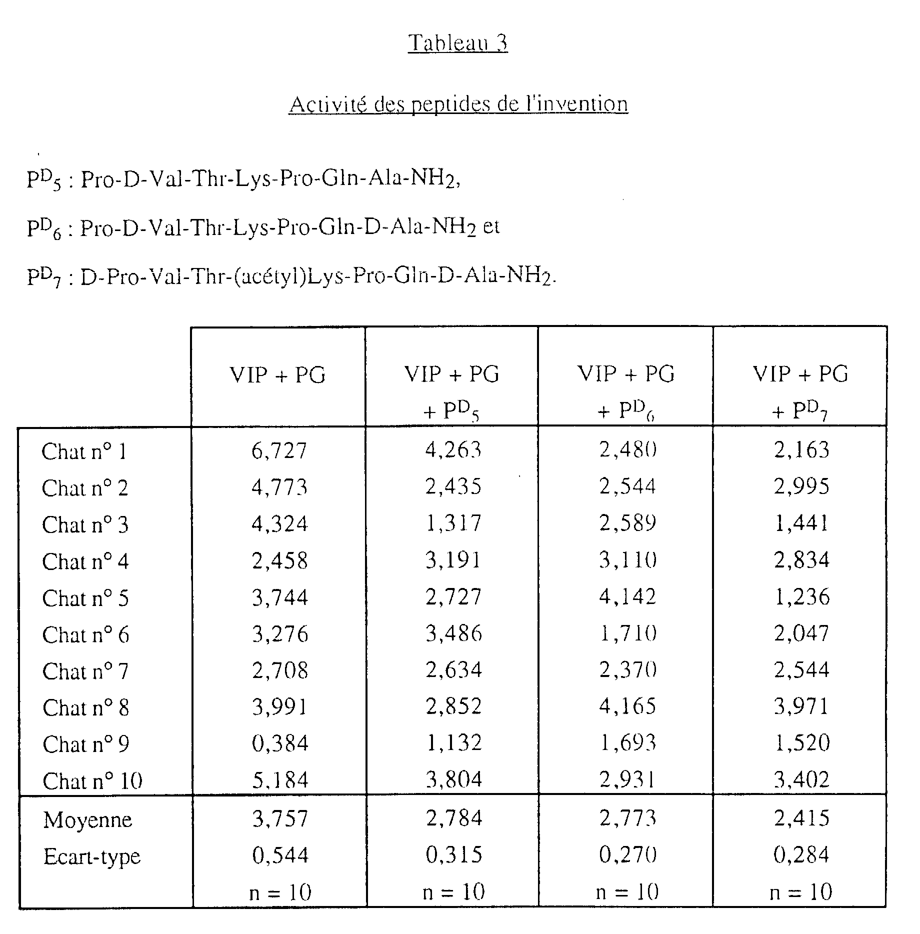

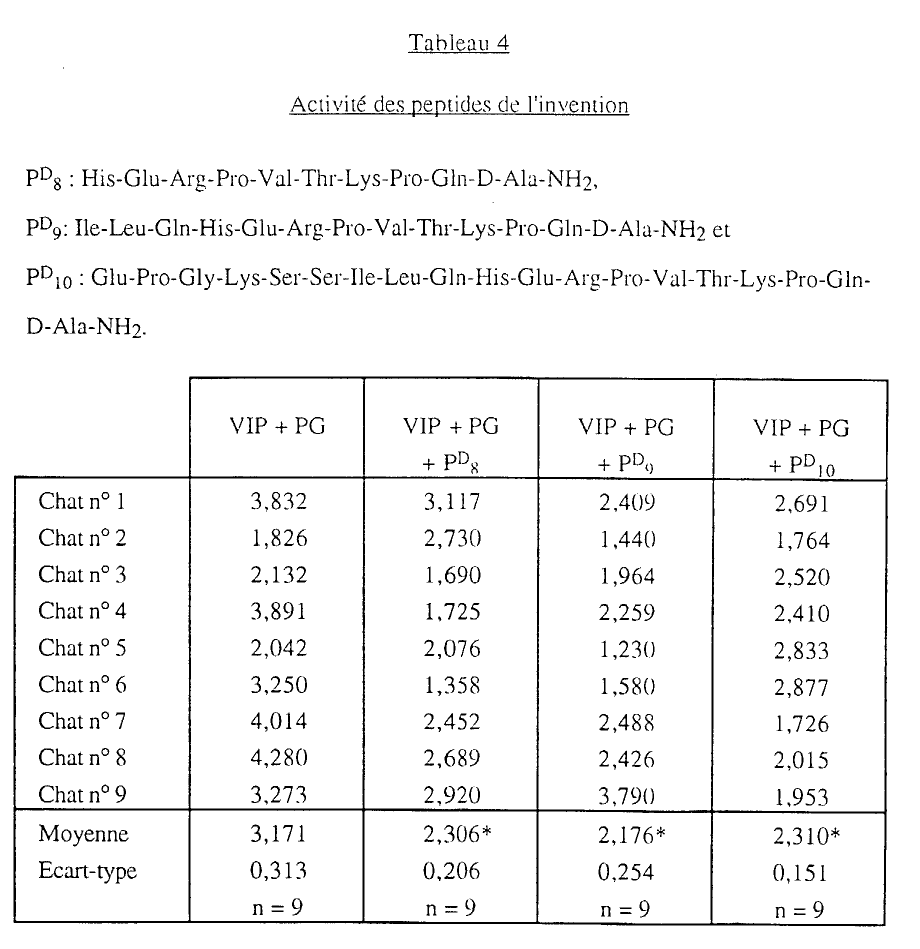

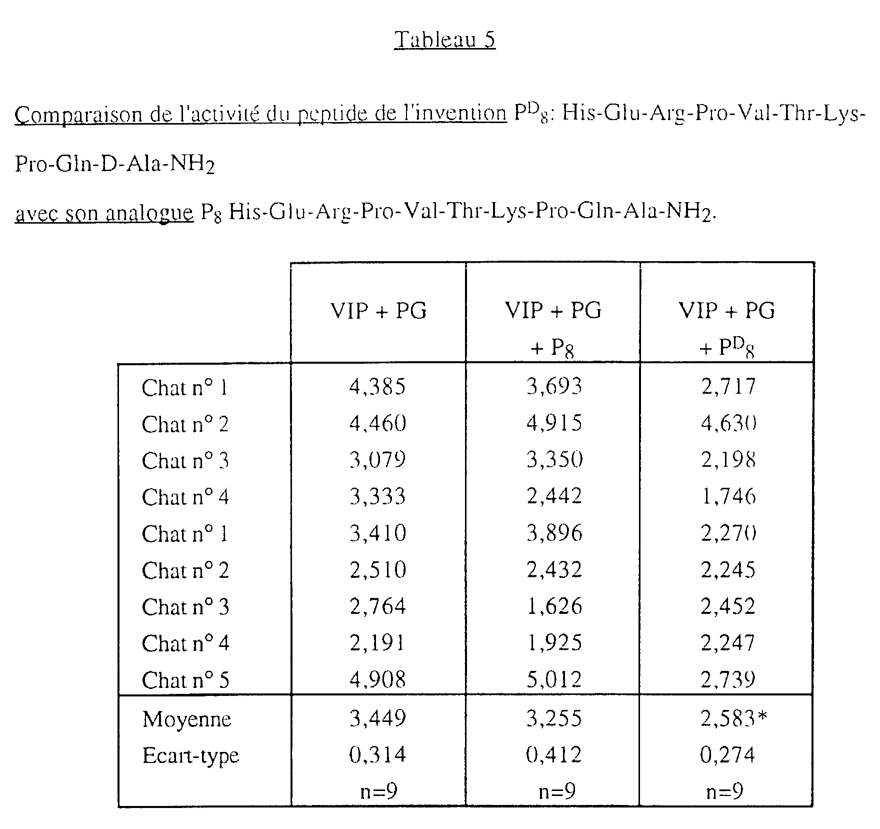

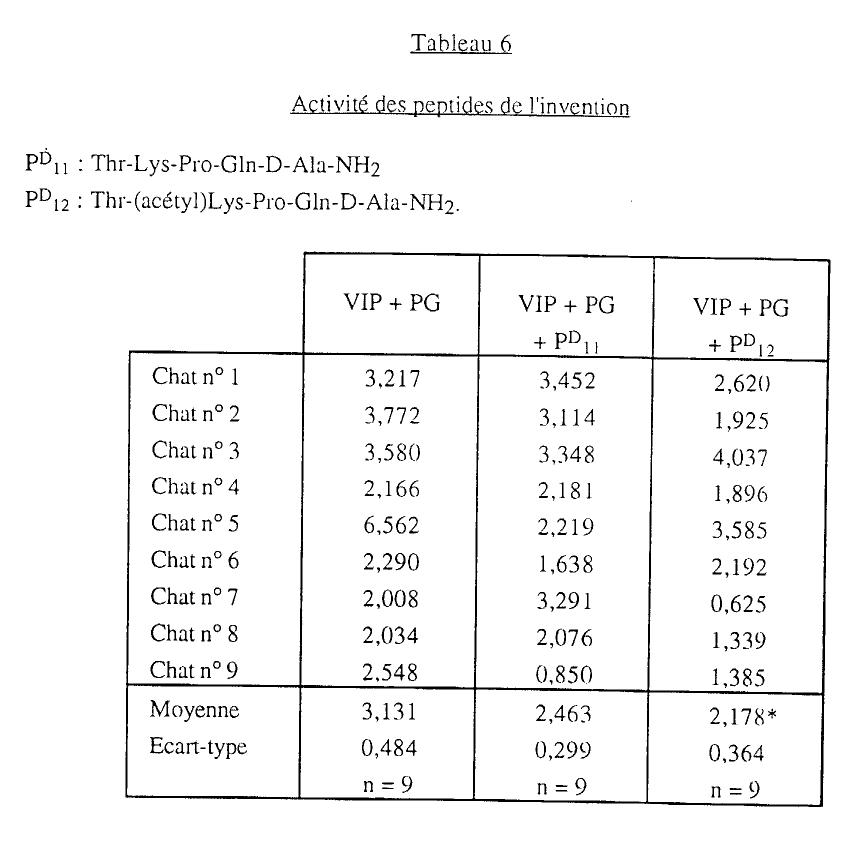

## Revendications

1. Peptide de formule générale I
A₁―A₂―A₃―X I
dans laquelle
A₁ représente le résidu L-Thr ou D-Thr ; ou l'une des séquences suivantes dans laquelle au moins un résidu d'acide aminé peut être de configuration D : 
Val-Thr,
Pro-Val-Thr,
Arg-Pro-Val-Thr,
Glu-Arg-Pro-Val-Thr,
His-Glu-Arg-Pro-Val-Thr,
Gln-His-Glu-Arg-Pro-Val-Thr,
Leu-Gln-His-Glu-Arg-Pro-Val-Thr,
Ile-Leu-Gln-His-Glu-Arg-Pro-Val-Thr,
Ser-Ile-Leu-Gln-His-Glu-Arg-Pro-Val-Thr,
Ser-Ser-Ile-Leu-Gln-His-Glu-Arg-Pro-Val-Thr,
Lys-Ser-Ser-Ile-Leu-Gln-His-Glu-Arg-Pro-Val-Thr,
Gly-Lys-Ser-Ser-Ile-Leu-Gln-His-Glu-Arg-Pro-Val-Thr,
Pro-Gly-Lys-Ser-Ser-Ile-Leu-Gln-His-Glu-Arg-Pro-Val-Thr ou
Glu-Pro-Gly-Lys-Ser-Ser-Ile-Leu-Gln-His-Glu-Arg-Pro-Val-Thr;
A₂ représente le résidu Lys-Pro-Gln-Ala dans laquelle au moins un résidu d'acide aminé peut être de configuration D;
A₃ représente une liaison covalente ou la séquence peptidique -Gly-A₄-A₅ dans laquelle A₄ et A₅ représentent, indépendamment, chacun, un résidu d'acide aminé basique ;
X représente le groupement hydroxy ; amino ; alkylamino, ledit peptide de formule I caractérisé en ce qu'il contient au moins un résidu d'acide aminé de configuration D.

2. Peptide contenant la séquence d'acide aminé A₁-A₂-A₃ dans laquelle A₁, A₂ et A₃ sont définis selon la revendication 1, le peptide contenant au moins un résidu d'acide aminé de configuration D.

3. Dérivé de substitution d'un peptide selon la revendication 1 ou 2 dans lequel un ou plusieurs résidus d'acide aminé est substitué par un ou des groupes protecteurs couramment utilisés dans la chimie des peptides à usage biologique; quand il y en a deux ou plus, ils ne sont pas nécessairement identiques.

4. Peptide selon la revendication 3 contenant au moins un résidu lysine protégé par le groupement acétyl.

5. Peptide selon l'une des revendications précédentes sous la forme de son sel pharmaceutiquement acceptable.

6. Peptide selon l'une des revendications 1 à 5, contenant un résidu d'acide aminé de configuration D.

7. Peptide selon la revendication 6, dont le résidu d'acide aminé de configuration D se situe sur l'extrémité C-terminale ou N-terminale.

8. Peptide selon la revendication 7, dont le résidu d'acide aminé de configurations D se situe sur l'extrémité C-terminale.

9. Peptide selon l'une des revendications 6 à 8, dans lequel A₂ représente Lys-Pro-Gln-D-Ala et A₃ représente une liaison covalente.

10. Peptide selon la revendication 9, de formule Thr-Lys-Pro-Gln-D-Ala-NH₂ ou Thr-(acétyl)Lys-Pro-Gln-D-Ala-NH₂.

11. Peptide selon la revendication 9, de formule Pro-Val-Thr-Lys-Pro-Gln-D-Ala-NH₂ ou Pro-Val-Thr-(acétyl)Lys-Pro-Gln-D-Ala-NH₂.

12. Peptide selon la revendication 9, de formule His-Glu-Arg-Pro-Val-Thr-Lys-Pro-Gln-D-Ala-NH₂.

13. Peptide selon la revendication 9, de formule Ile-Leu-Gln-His-Glu-Arg-Pro-Val-Thr-Lys-Pro-Gln-D-Ala-NH₂.

14. Peptide selon la revendication 9, de formule Glu-Pro-Gly-Lys-Ser-Ser-Ile-Leu-Gln-His-Glu-Arg-Pro-Val-Thr-Lys-Pro-Gln-D-Ala-NH₂.

15. Peptide selon la revendication 7, dont le résidu d'acide aminé de configuration D se situe sur l'extrémité N-terminale.

16. Peptide selon la revendication 15, dans lequel A₁ représente D-Thr ou une séquence telle que définie ci-dessus dans laquelle le résidu d'acide aminé en position N-terminale de cette séquence est de configuration D.

17. Peptide selon la revendication 16, de formule D-Pro-Val-Thr-Lys-Pro-Gln-Ala-NH₂.

18. Peptide selon l'une des revendications là 5. contenant deux résidus d'acide aminé de configuration D.

19. Peptide selon la revendication 18, dont un résidu d'acide aminé de configuration D se situe en position C-terminale

20. Peptide selon l'une des revendications 18 à 19, dont le deuxième résidu d'acide aminé de configuration D se situe en position N-terminale du peptide.

21. Peptide selon la revendication 20, de formule D-Pro-Val-Thr-Lys-Pro-Gln-D-Ala-NH₂.

22. Peptide selon la revendication 20, de formule D-Pro-Val-Thr-(acétyl)Lys-Pro-Gln-D-Ala-NH₂.

23. Compositions pharmaceutiques contenant, à titre de principe actif, une quantité efficace d'au moins un peptide selon l'une des revendications 1 à 22, en association avec un diluent ou support pharmaceutiquement acceptable.

## Patentansprüche

1. Peptid mit der allgemeinen Formel I
A₁―A₂―A₃―X I
in der
A₁ für den Rest L-Thr oder D-Thr bzw, eine der nachfolgenden Sequenzen steht, in der wenigstens ein Aminosäurerest die Konfiguration D haben kann : 
Val-Thr,
Pro-Val-Thr,
Arg-Pro-Val-Thr,
Glu-Arg-Pro-Val-Thr,
His-Glu-Arg-Pro-Val-Thr,
Gln-His-Glu-Arg-Pro-Val-Thr,
Leu-Gln-His-Glu-Arg-Pro-Val-Thr,
Ile-Leu-Gln-His-Glu-Arg-Pro-Val-Thr,
Ser-Ile-Leu-Gln-His-Glu-Arg-Pro-Val-Thr,
Ser-Ser-Ile-Leu-Gln-His-Glu-Arg-Pro-Val-Thr,
Lys-Ser-Ser-Ile-Leu-Gln-His-Glu-Arg-Pro-Val-Thr,
Gly-Lys-Ser-Ser-Ile-Leu-Gln-His-Glu-Arg-Pro-Val-Thr,
Pro-Gly-Lys-Ser-Ser-Ile-Leu-Gln-His-Glu-Arg-Pro-Val-Thr oder
Glu-Pro-Gly-Lys-Ser-Ser-Ile-Leu-Gln-His-Glu-Arg-Pro-Val-Thr ;
A₂ den Rest Lys-Pro-Gln-Ala darstellt, in dem wenigstens ein Aminosäurerest die Konfiguration D haben kann ;
A₃ eine kovalente Bindung oder die Peptidsequenz Gly-A₄-A₅ darstellt, in der A₄ und A₅ jeder für sich einen basischen Aminosäurerest darstellen ;
X die Hydroxyl-, Amino-, Alkylamino-Gruppe darstellt, dadurch gekennzeichnet, daß das besagte Peptid gemäß Formel I wenigstens einen Aminosäurerest der Konfiguration D enthält.

2. Peptid, das die Aminosäuresequenz A₁-A₂-A₃ enthält, in der A₁, A₂ und A₃ der Definition gemäß Anspruch 1 entsprechen, wobei das Peptid wenigstens einen Aminosäurerest der Konfiguration D enthält.

3. Substitutionsderivat eines Peptids gemäß Anspruch 1 oder 2, in dem ein bzw. mehrere Aminosäurereste durch eine bzw. mehrere Schutzgruppen ersetzt worden sind, wie sie gegenwärtig in der Chemie der Peptide für biologische Zwecke verwendet werden ; handelt es sich dabei um zwei oder mehrere, so sind diese nicht unbedingt miteinander identisch.

4. Peptid gemäß Anspruch 3 mit wenigstens einem durch die Acetylgruppe geschützten Lysinrest.

5. Peptid gemäß einem der vorhergehenden Ansprüche in der Form seines pharmazeutisch akzeptierbaren Salzes.

6. Peptid gemäß einem der Ansprüche 1 bis 5 mit einem Aminosäurerest der Konfiguration D.

7. Peptid gemäß Anspruch 6, bei dem der Aminosäurerest der Konfiguration D am endständigen C oder am endständigen N anliegt.

8. Peptid gemäß Anspruch 7, bei dem der Aminosäurerest der Konfiguration D am endständigen C anliegt.

9. Peptid gemäß einem der Ansprüche 6 bis 8, bei dem A₂ für Lys-Pro-Gln-D-Ala steht und A₃ eine kovalente Bindung darstellt.

10. Peptid gemäß Anspruch 9 mit der Formel Thr-Lys-Pro-Gln-D-Ala-NH₂ oder Thr-(acetyl)Lys-Pro-Gln-D-Ala-NH₂.

11. Peptid gemäß Anspruch 9 mit der Formel Pro-Val-Thr-Lys-Pro-Gln-D-Ala-NH₂ oder Pro-Val-Thr-(acetyl)Lys-Pro-Gln-D-Ala-NH₂.

12. Peptid gemäß Anspruch 9 mit der Formel His-Glu-Arg-Pro-Val-Thr-Lys-Pro-Gln-D-Ala-NH₂.

13. Peptid gemäß Anspruch 9 mit der Formel Ile-Leu-Gln-His-Glu-Arg-Pro-Val-Thr-Lys-Pro-Gln-D-Ala-NH₂.

14. Peptid gemäß Anspruch 9 mit der Formel Glu-Pro-Gly-Lys-Ser-Ser-Ile-Leu-Gln-His-Glu-Arg-Pro-Val-Thr-Lys-Pro-Gln-D-Ala-NH₂.

15. Peptid gemäß Anspruch 7, bei dem der Aminosäurerest der Konfiguration D am endständigen N anliegt.

16. Peptid gemäß Anspruch 15, bei dem A₁ für D-Thr bzw. eine wie oben definierte Sequenz steht, bei der der am endständigen N dieser Sequenz anliegende Aminosäurerest die Konfiguration D hat.

17. Peptid gemäß Anspruch 16 mit der Formel D-Pro-Val-Thr-Lys-Pro-Gln-Ala-NH₂.

18. Peptid gemäß einem der Ansprüche 1 bis 5, das zwei Aminosäurereste der Konfiguration D enthält.

19. Peptid gemäß Anspruch 18, bei dem ein Aminosäurerest der Konfiguration D am endständigen C anliegt.

20. Peptid gemäß einem der Ansprüche 18 bis 19, bei dem der zweite Aminosäurerest der Konfiguration D am endständigen N des Peptids anliegt.

21. Peptid gemäß Anspruch 20 mit der Formel D-Pro-Val-Thr-Lys-Pro-Gln-D-Ala-NH₂.

22. Peptid gemäß Anspruch 20 mit der Formel D-Pro-Val-Thr-(acetyl)Lys-Pro-Gln-D-Ala-NH₂.

23. Arzneimittel, die als Hauptwirkstoff eine wirksame Menge wenigstens eines Peptides gemäß einem der Ansprüche 1 bis 22 zusammen mit einem pharmazeutisch akzeptierbaren Verdünnungsmittel oder einer pharmazeutisch akzeptierbaren Trägersubstanz enthalten.

## Claims

1. Peptide of general formula I
A₁―A₂―A₃―X I
in which
A₁ represents the L-Thr or D-Thr residue or one of the following sequences in which at least one amino acid residue can be of D configuration 
Val-Thr,
Pro-Val-Thr,
Arg-Pro-Val-Thr,
Glu-Arg-Pro-Val-Thr,
His-Glu-Arg-Pro-Val-Thr,
Gln-His-Glu-Arg-Pro-Val-Thr,
Leu-Gln-His-Glu-Arg-Pro-Val-Thr,
Ile-Leu-Gln-His-Glu-Arg-Pro-Val-Thr,
Ser-Ile-Leu-Gln-His-Glu-Arg-Pro-Val-Thr,
Ser-Ser-Ile-Leu-Gln-His-Glu-Arg-Pro-Val-Thr,
Lys-Ser-Ser-Ile-Leu-Gln-His-Glu-Arg-Pro-Val-Thr,
Gly-Lys-Ser-Ser-Ile-Leu-Gln-His-Glu-Arg-Pro-Val-Thr,
Pro-Gly-Lys-Ser-Ser-Ile-Leu-Gln-His-Glu-Arg-Pro-Val-Thr or
Glu-Pro-Gly-Lys-Ser-Ser-Ile-Leu-Gln-His-Glu-Arg-Pro-Val-Thr ;
A₂ represents the Lys-Pro-Gln-Ala residue in which at least one amino acid residue can be of D configuration ;
A₃ represents a covalent bond or the peptide sequence Gly-A₄-A₅ in which A₄ and A₅ each represent, independently, a basic amino acid residue ;
X represents the hydroxy, amino, alkylamino group, said peptide of general formula I characterized in that it contains at least one amino acid residue of D configuration.

2. Peptide containing the amino acid sequence A₁-A₂-A₃ in which A₁, A₂ and A₃ are defined according to claim 1, the peptide containing at least one amino acid residue of D configuration.

3. Substitution derivative of a peptide according to claim 1 or 2 in which one or more amino acid residues is substituted by one or more protective groups commonly used in the chemistry of peptides for biological uses ; when there are two or more, they are not necessarily identical.

4. Peptide according to claim 3 containing at least one lysine residue protected by the acetyl group.

5. Peptide according to one of the preceding claims in the form of its pharmaceutically acceptable salt.

6. Peptide according to one of claims 1 to 5, containing an amino acid residue of D configuration.

7. Peptide according to claim 6, of which the amino acid residue of D configuration is found on the C-terminal or N-terminal end.

8. Peptide according to claim 7, of which the amino acid residue of D configuration is found on the C-terminal end.

9. Peptide according to one of claims 6 to 8, in which A₂ represents Lys-Pro-Gln-D-Ala and A₃ represents a covalent bond.

10. Peptide according to claim 9, of formula Thr-Lys-Pro-Gln-D-Ala-NH₂ or Thr-(acetyl)Lys-Pro-Gln-D-Ala-NH₂.

11. Peptide according to claim 9, of formula Pro-Val-Thr-Lys-Pro-Gln-D-Ala-NH₂ or Pro-Val-Thr-(acetyl)Lys-Pro-Gln-D-Ala-NH₂.

12. Peptide according to claim 9, of formula His-Glu-Arg-Pro-Val-Thr-Lys-Pro-Gln-D-Ala-NH₂.

13. Peptide according to claim 9, of formula Ile-Leu-Gln-His-Glu-Arg-Pro-Val-Thr-Lys-Pro-Gln-D-Ala-NH₂.

14. Peptide according to claim 9, of formula Glu-Pro-Gly-Lys-Ser-Ser-Ile-Leu-Gln-His-Glu-Arg-Pro-Val-Thr-Lys-Pro-Gln-D-Ala-NH₂.

15. Peptide according to claim 7, of which the amino acid residue of D configuration is found on the N-terminal end.

16. Peptide according to claim 15, in which A₁ represents D-Thr or a sequence as defined above in which the amino acid residue in the N-terminal position of this sequence is of D configuration.

17. Peptide according to claim 16, of formula D-Pro-Val-Thr-Lys-Pro-Gln-Ala-NH₂.

18. Peptide according to one of claims 1 to 5, containing two amino acid residues of D configuration.

19. Peptide according to claim 18, of which one amino acid residue of D configuration is found in the C-terminal position.

20. Peptide according to claims 18 to 19, of which the second amino acid residue of D configuration is found in the N-terminal position of the peptide.

21. Peptide according to claim 20, of formula D-Pro-Val-Thr-Lys-Pro-Gln-D-Ala-NH₂.

22. Peptide according to claim 20, of formula D-Pro-Val-Thr-(acetyl)Lys-Pro-Gln-D-Ala-NH₂.

23. Pharmaceutical compositions containing, as active ingredient, an effective quantity of at least one peptide according to one of claims 1 to 22, in combination with a pharmaceutically acceptable diluent or support.
